(19) Europäisches Patentamt

European Patent Office

Office européen des brevets

(11) Publication number: **0 513 510 A1**

## (12) EUROPEAN PATENT APPLICATION

(21) Application number: **92105598.4**

(22) Date of filing: **01.04.92**

(51) Int. Cl.⁵: **A61K 49/02**, A61K 43/00

(30) Priority: **01.05.91 US 694174**

(43) Date of publication of application:
**19.11.92 Bulletin 92/47**

(84) Designated Contracting States:
**AT BE CH DE DK ES FR GB GR IT LI LU MC
NL PT SE**

(71) Applicant: **E.R. SOUIBB & SONS, INC.
P.O.Box 4000
Princeton New Jersey 08543-4000(US)**

(72) Inventor: **Tu, Jan-I
12 LeParc Court
Lawrenceville, NJ 08648(US)**
Inventor: **Yost, Fred
3 Ouail Run
Long Valley, NJ 07853(US)**
Inventor: **Wen, Mei-Li
6 Castle Court
North Brunswick, NJ 08902(US)**
Inventor: **Jagoda, Elaine
965 Bergen Avenue
North Brunswick, NJ 08902(US)**

(74) Representative: **Josif, Albert et al
Baaderstrasse 3
W-8000 München 5(DE)**

(54) Method for stabilizing radiolabeled biological molecules using ion exchange resins.

㊳ A method for the stabilization of radiolabeled biological molecules using an ion exchange resin.

Radiolabeled biological molecules are finding increasing use as diagnostic and therapeutic agents. For example, radiolabeled monoclonal antibodies have been shown to cause tumor shrinkage. In addition, radiolabeled monoclonal antibodies have been utilized in diagnostic imaging. However, the stability of radiolabeled biological molecules has continued to be a problem. Various approaches have been used to stabilize these molecules. For example, freezing of the radiolabeled biological molecules at -70°C has been employed.

However, there is a continued need for new methods for the stabilization of radiolabeled biological molecules.

The present invention concerns a method for the stabilization of radiolabeled biological molecules using ion exchange resins.

In particular, the present invention concerns a method for the stabilization of a radiolabeled biological molecule comprising contacting the radiolabeled biological molecule with an ion exchange resin and allowing the radiolabeled biological molecule to remain in contact with the ion exchange resin to stabilize the radiolabeled biological molecule.

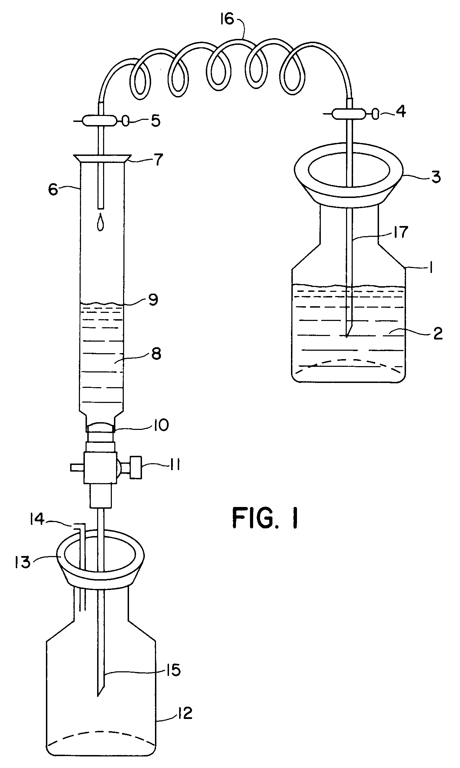

Figure 1 shows an apparatus which can be used for the stabilization of radiolabeled biological molecules using ion exchange resins.

The present invention concerns a method for the stabilization of radiolabeled biological molecules using an ion exchange resin.

In particular, the present invention concerns a method for the stabilization of a radiolabeled biological molecule comprising contacting the radiolabeled biological molecule with an ion exchange resin and allowing the radiolabeled biological molecule to remain in contact with the ion exchange resin to stabilize the radiolabeled biological molecule.

Various radiolabeled biological molecules may be stabilized in accordance with the present invention. For example, radiolabeled nucleic acid molecules (e.g., ribonucleic acid [RNA] and deoxyribonucleic acid [DNA] molecules), peptides, polypeptides, proteins, hormones, and polyclonal and monoclonal antibodies and fragments thereof may be so stabilized. A preferred radiolabeled biological molecule for stabilization is a radiolabeled monoclonal antibody.

Various radiolabels may be stabilized in accordance with the present invention. For example, $^{125}$I, $^{131}$I and other beta emitters such as $^{90}$Y or $^{64}$Cu, and other alpha emitters such as $^{212}$Bi may be so stabilized.

Depending on the specific radiolabeled biological molecule to be stabilized, the ion exchange resin can be either an anion exchange resin or a cation exchange resin. A cation exchange resin is employed when the charge on the biological molecule is such that the molecule will not interact with the cationic groups on the resin. An anion exchange resin is employed when the charge on the biological molecule is such that the molecule will not interact with anionic groups on the resin. A preferred ion exchange resin is the anion exchange resin, DEAE-Sephadex (Pharmacia-LKB Biotechnology Inc., Piscataway, NJ 08855). Other suitable ion exchange resins include, for example, L11 DE 52(Whatman Lab Sales, Inc., Hillsboro, OR 97123) and carboxymethyl cellulose (Bio-Rad Laboratories, Richmond, CA).

In practicing the method of the present, the radiolabeled biological molecule must first be contacted with the ion exchange resin. This can be done in various ways. For example, the radiolabeled biological molecule may be dissolved in an appropriate buffer solution and then contacted with the ion exchange resin. Various buffers, for example, phosphate buffered saline (PBS; 10 mM phosphate, pH 7.2, 0.15 M NaCl), TRIS [Tris(hydroxymethyl)aminomethane), and HEPES (N-[2-hydroxyethyl] piperazine-N'-[2-ethanesulfonic acid]) may be employed. The concentration of the buffer solution can range from about 10 to about 50 mM, while the pH of the buffer solution can range from about 6.5 to about 7.5. In general, the concentration and pH of the buffer is chosen so that the biological molecule will not bind to the ion exchange resin. The temperature of the buffer solution should range from about -70°C to about 40°C. The buffer solution may also contain other materials. For example, human serum albumin (HSA) may be added in concentrations ranging from about 1% to about 5% as an additional stabilizing agent. Other materials which may be added to the buffer solution include, for example, mannitol or reducing agents. The volume of buffer used to dissolve the radiolabeled biological molecule should be minimized. In general, the volume of buffer used should range from about 50% to about 90% of the volume of the ion exchange resin, although a buffer volume equivalent to about 80% of the volume of the ion exchange resin is preferred.

Once the radiolabeled biological molecule has been contacted with the ion exchange resin, it may be stored until the radiolabeled biological material is no longer useful for its intended purpose. For example, a radioiodinated monoclonal antibody such as $^{131}$I ChiL6 may be stored until the radiolabel decays to the extent that the radiolabeled antibody can no longer be used therapeutically. Storage temperatures ranging from about -70°C to about 4°C may be employed, although a storage temperature of about 4°C is preferred.

The radiolabeled biological material must of course be recovered from the ion exchange resin before it can be used. For example, if the radiolabeled biological molecule and ion exchange resin are contained in a column, the radiolabeled biological molecule may be eluted from the column using a buffer solution. In general, the same buffer used to dissolve the radiolabeled biological molecule (see discussion above) is employed, although a different buffer solution can also be used.

An apparatus which can be used to practice the method of the present invention is shown in Figure 1 and compromises a column device 6, an elution buffer vial 1 and a collection vial 12. Referring now to Figure 1, the column device 6 contains an ion exchange resin 8 in contact with the radiolabeled biological molecule interposed between a coarse filter 9 and a 0.22 $\mu$M filter 10. The entrance to column device 6 is closed with a seal 7 and a conventional stopcock 5, and the exit of column device 6 is closed with a conventional stopcock 11. The elution buffer vial 1 is pressurized and contains elution buffer 2, and is sealed with an aluminum crimped rubber stopper 13. The collection vial 12 is sealed with an aluminum crimped rubber stopper 13 containing an air vent 14. In order to elute the radiolabeled biological molecule from the ion exchange resin 8, the following procedure can be used:

1. Screw a needle 15 (e.g., a 21 gauge matal needle) onto stopcock 11 of column device 6;

2. Insert the needle 15 through the aluminum crimped rubber stopper 13 so that it extends into the collection vial 12;

3. Insert a needle 17 connected to a stopcock 4 through the aluminum crimped rubber stopper 3 so that it extends into the elution buffer vial 1;

4. Connect the column device 6 to the elution buffer vial 1 by extending tubing 16 between stopcock 4 and stopcock 5; and

5. Open stopcocks 4, 5, 11 in the sequence 11, 5 and 4.

Although no particular mechanism of action is asserted for the present invention, the possible functions of the ion exchange resin are: (1) as a scavenger of free radiolabel which is liberated from the radiolabeled biological molecule; (2) as a potential stabilizer for the radiolabeled biological molecule. The radiolabeled biological molecule will be evenly dispersed within the numerous cavities of the matrix of the ion exchange resin. This dispersion may decrease the interaction between radiolabeled biological molecules which may ultimately decrease the radioactive bombardment among the radiolabeled molecules; (3) as a scavenger of free radicals. This may minimize the damage to radiolabeled biological molecules caused by free radicals.

The following examples are further illustrative of the present invention. These examples are not intended to limit the scope of the present invention, and provide further understanding of the invention.

Example 1

Stabilization of $^{131}$I-Chimeric L6 Monoclonal Antibody

Chimeric L6 antibody (ChiL6) is a monoclonal antibody developed from MuL6, a murine monoclonal antibody. MuL6 recognizes a membrane antigen (L6) found in human lung, breast and colon carcinoma. In order to form ChiL6, the constant regions of MuL6 were substituted with the constant regions from human IgG1. The resulting chimeric L6 antibody is about 70% human origin and about 30% murine origin. ChiL6 labeled with Na[131]I has been shown to have good tumor uptake with little bone marrow damage in a human with breast cancer. It has been shown that this antibody also binds to tumors in the thyroid, lung, prostate, ovary and colon.

In order to stabilize $^{131}$I-ChiL6 with an ion exchange resin, samples of $^{131}$I-ChiL6 were prepared in phosphate buffered saline with different concentrations of human serum albumin (HSA), with and without DEAE-Sephadex, and stored at 4°C or -70°C in capped tubes. The concentration of the $^{131}$I-ChiL6 ranged from 0.5 to 1.5 mg/ml and 5 to 15 mci/ml. In order to monitor the stability of the $^{131}$I-ChiL6, two assays were used.

First, the radiochemical purity (RCP) of the $^{131}$I-ChiL6 was analyzed by HPLC using a TSK 3000 SW gel exclusion column (Phenomenex, Torrance, CA). The column was developed isocratically with phosphate buffered saline, pH 7.2 at a flow rate of 1 ml/min.

Second, immunoreactivity of the $^{131}$I-ChiL6 antibody was measured using an affinity column prepared from partially purified membrane preparations of H3347 cells (Bristol-Myers Squibb, Oncogene Division, Seattle, WA), which express antigen for L6 antibody [See, Liu, A.Y. et al., Proc. Natl. Acad. Sci. USA 84, 3439-3443 (1987)]. Membrane preparations derived from tumors and other cell lines which express antigen for L6 antibody can also be used for this purpose [See, Hellstrom, I. et al., Proc. Natl. Acad. Sci. USA 83, 7059-7063 (1986)]. Membrane preparations were prepared as follows. H3347 cells from flasks or roller bottles were harvested with standard EDTA solution and washed with PBS. Cells were used immediately or quick frozen in liquid nitrogen and stored at -70°C (frozen cells were thawed at 37°C before further use). Cells were suspended in 10 mM Tris-HCl pH 7.4, 500 uM PMSF, 10 ug/ml aprotinin using 4 ml of buffer per ml of packed cells, and cooled on ice for 15 minutes. Cells were then dounce homogenized with 50 strokes of a Dounce homogenizer. Alternatively, a power homogenizer may be used. The lysate was centrifuged at 2500 xg for 5 minutes to remove nuclei and debris, and the supernatant solution was transferred to a new tube and centrifuged at 12,000 xg for 30 minutes. The membranes were located in the pellet. The membrane pellets were suspended in PBS containing 500 $\mu$M PMSF and 10 $\mu$g/ml aprotinin, and the the protein concentration was measured using the Bradford assay. The membrane suspensions were frozen and held at -70°C for storage. These membrane preparations were then covalently bound to a Reacti-gel agarose matrix (Pierce Chemical Co., Rockford, IL 61105) to prepare the affinity column. H3347 cell membrane preparations were dialyzed against 0.1 M sodium borate (pH 8.5) at room temperature for 6 to 8 hours with two buffer changes. The Reacti-gel agarose matrix was filtered on Whatman #1 filter paper and washed five times with two volumes of 0.1 M sodium borate (pH 8.5). The H3347 cell membrane preparations were added to the Reacti-gel at a concentration of 1 mg of cell membrane per ml of gel (packed volume), and incubated at 4°C overnight with slow stirring. The H3347 cell membrane coupled gel was washed with borate buffer, resuspended in borate buffer containing 1% ethanolamine (2-Aminoethanol) to block the remaining active sites, and incubated at 4°C overnight with slow stirring. The resulting H3347 Reacti-gel affinity matrix was washed with TBS buffer (20 mM Tris HCl, 150 mM NaCl, pH 7.2) and stored at 4°C until use. A 2.5 ml H3347 Reacti-gel affinity column was prepared by packing a Rainin Leur-Lock column with the affinity matrix. The column was stored in PBS buffer containing 0.02% NaN$_3$.

To determine immunoreactivity using this affinity column, approximately 0.1 $\mu$Ci of labeled antibody was loaded onto the affinity column. The antibody was washed in with 0.5 ml of 10 mM phosphate buffer, 150 mM NaCl pH 7.2 (PBS) and incubated for 45 minutes. The column was washed with a standard amount (about 15 ml) of PBS buffer followed by a standard amount (about 6 ml) of 6M guanidine-HCl. All eluant from this column was collected using fraction collectors and all tubes were counted. The immunoreactivity was determined by the radioactive counts collected by the addition of guanidine-HCl divided by the total counts eluted from the column times 100 and reported as a percentage.

An optimized affinity column procedure for the H3347 affinity column involves the following steps: (1) wash column with 10 ml of PBS buffer containing 1% HSA; (2) apply 100,000 cpm of radiolabeled L6 onto column, collect effluent; (3) wash column with 0.5 ml PBS buffer, collect effluent; (4) apply 0.5 ml of PBS buffer onto column and incubate the column at room temperature for 45 minutes; (5) wash column with 15 ml PBS buffer, collect 10x1 ml fractions; (6) elute antibody with 6 ml 6M Guanidine HCl, collect 6x1 ml fractions; (7) wash column with 9 ml PBS buffer, collect 9x1 ml fractions; (8) store column in PBS buffer containing NaN$_3$; and (9) calculate % binding, wherein % binding = cpm eluted with Guanidine/Total cpm recovered.

Using the above techniques, the stability of the $^{131}$I-ChiL6 antibody at 4°C in the presence of DEAE-Sephadex and various concentrations of HSA (1%, 2% and 5%), with or without DEAE-Sephadex, was compared with the stability of the $^{131}$I-ChiL6 antibody at -70°C in the presence of 1% HSA but not DEAE-Sephadex. As can be seen from Table 1, the $^{131}$I-ChiL6 antibodies were stabilized to a significant degree by the DEAE-Sephadex, with the samples containing 5% HSA and DEAE-Sephadex exhibiting the greatest stability after seven days, this stability being comparable to the stability observed at -70°C after seven days. The stability of $^{125}$I-ChiL6 was also examined in the presence of various amounts of DEAE-Sephadex. Specifically, DEAE-Sephadex was added to a $^{125}$I-ChiL6 solution (specific activity of 13.9 mCi/mg) containing 1% HSA, at antibody to packed gel ratios of 1:0.5 and 1:1 (v:v). As can be seen from Table 2, both concentrations of DEAE-Sephadex stabilized the $^{125}$I-ChiL6 antibody.

The stability of DEAE-Sephadex to stabilize high specific activity $^{131}$I-ChiL6 (Table 3) and moderate specific activity $^{131}$I-ChiL6 (Table 4) was also examined. It can be seen that DEAE-Sephadex was able to stabilize both high and moderate specific activity $^{131}$I-ChiL6, with samples which received 2+ volumes of DEAE-Sephadex exhibiting the greatest increase in stability at 4°C.

# TABLE 1. STABILTY STUDY OF $^{131}$I-ChiL6

(Specific Activity = 7.3 mCi/mg,
Radioconcentration = 7.3 mCi/mL)

| Day Post-labeling | -70°C No Addition of DEAE % Binding HSA 1% | -70°C No Addition of DEAE RCP (%) HSA 1% | 4°C No Addition of DEAE % Binding HSA 1% | 4°C No Addition of DEAE % Binding HSA 2% | 4°C No Addition of DEAE % Binding HSA 5% | 4°C No Addition of DEAE RCP (%) HSA 1% | 4°C No Addition of DEAE RCP (%) HSA 2% | 4°C No Addition of DEAE RCP (%) HSA 5% | 4°C With DEAE % Binding HSA 1% | 4°C With DEAE % Binding HSA 2% | 4°C With DEAE % Binding HSA 5% | 4°C With DEAE RCP (%) HSA 1% | 4°C With DEAE RCP (%) HSA 2% | 4°C With DEAE RCP (%) HSA 5% |
|---|---|---|---|---|---|---|---|---|---|---|---|---|---|---|
| 0 | 89.2 | 100.0 | 89.2 | 89.2 | 89.2 | 100.0 | 100.0 | 100.0 | 89.2 | 89.2 | 89.2 | 100.0 | 100.0 | 100.0 |
| 1 | -- | -- | 75.1 | 82.4 | 91.9 | 87.6 | 93.8 | 95.0 | 80.6 | 88.4 | 91.9 | 100.0 | 100.0 | 100.0 |
| 3 | 86.0 | 97.2 | -- | -- | -- | -- | -- | -- | -- | -- | -- | -- | -- | -- |
| 4 | -- | -- | 50.8 | 70.4 | 79.5 | 80.2 | 88.2 | 90.8 | 83.6 | 86.4 | 89.4 | 100.0 | 100.0 | 100.0 |
| 7 | 83.5 | 97.4 | 40.8 | 59.6 | 77.0 | 85.0 | 85.8 | 88.4 | 68.3 | 84.2 | 85.0 | 100.0 | 100.0 | 100.0 |

EP 0 513 510 A1

EP 0 513 510 A1

### Table 2

#### Summary of Stability Study of $^{125}$I-ChiL6 With and Without DEAE-Sephadex

(Specific Activity = 13.9 mCi/mg,
Radioconcentration = 5.8 mCi/mL)

| Day Post-Labeling | % Binding -70°C | | | RCP (%) -70°C | | | % Binding 4°C | RCP (%) 4°C |
|---|---|---|---|---|---|---|---|---|
| | Control | 1x DEAE | 2x DEAE | Control | 1x DEAE | 2x DEAE | Control | Control |
| 0 | 94.5 | 94.5 | 94.5 | 99.9 | 99.9 | 99.9 | 94.5 | 99.5 |
| 3 | 86.9 | 89.5 | 89.8 | 93.3 | 99.8 | 99.9 | 92.8 | 98.8 |
| 7 | 76.7 | 85.6 | 85.3 | 87.0 | 97.3 | 99.0 | 88.8 | 95.4 |

## Table 3

Stability Study of $^{131}$I-ChiL6

Specific Activity = 20.25 mCi/mg,
Radioconcentration = 7.95 mCi/mL)

| | -70°C | | | | 4°C | | | | | |
| | % Binding | | RCP (%) | | % Binding | | | RCP (%) | | |
| Day Post-Labeling | Control | 1x DEAE | Control | 1x DEAE | Control | 1x DEAE | 2x DEAE | Control | 1x DEAE | 2x DEAE |
|---|---|---|---|---|---|---|---|---|---|---|
| 0 | 78.3 | 78.3 | 95.0 | 95.0 | 78.3 | 78.3 | 78.3 | 95.0 | 95.0 | 95.0 |
| 3 | -- | -- | -- | -- | 30.8 | 51.1 | 63.8 | 62.4 | 77.5 | 85.5 |
| 4 | 80.0 | 81.2 | 95.0 | 97.9 | -- | -- | -- | -- | -- | -- |

EP 0 513 510 A1

Table 4

Stability study of $^{131}$I-ChiL6

(Specific Activity = 12.4 mCi/mg, Radioconcentration = 9.9 mCi/mL)

| Day Post-Labeling | -70°C | | | | 4°C | | | | | |
|---|---|---|---|---|---|---|---|---|---|---|
| | % Binding | | RCP (%) | | % Binding | | | RCP (%) | | |
| | Control | 1x DEAE | Control | 1x DEAE | Control | 1x DEAE | 2x DEAE | Control | 1x DEAE | 2x DEAE |
| 0 | 92.5 | 92.5 | 100.0 | 100.0 | 92.5 | 92.5 | 92.5 | 100.0 | 100.0 | 100.0 |
| 1 | -- | -- | -- | -- | 81.2 | 84.7 | 82.2 | 92.9 | 99.3 | 99.2 |
| 2 | 89.5 | 90.2 | 99.2 | 100.0 | -- | -- | -- | -- | -- | -- |
| 6 | -- | -- | -- | -- | 53.8 | 57.9 | 67.0 | 79.8 | 84.0 | 89.8 |
| 8 | 86.2 | 89.9 | 96.6 | 100.0 | -- | -- | -- | -- | -- | -- |

## Claims

1. A method for the stabilization of a radiolabeled biological molecule comprising contacting the radiolabeled biological molecule with an ion exchange resin and allowing the radiolabeled biological molecule to remain in contact with the ion exchange resin to stabilize the radiolabeled biological molecule.

2. The method according to Claim 1 wherein the radiolabeled biological molecule is a radiolabeled

8

antibody or fragment thereof.

3. The method according to Claim 2 wherein the radiolabeled antibody is a radiolabeled monoclonal antibody.

4. The method according to Claim 1 wherein the radiolabeled biological molecule is selected from the group consisting of a radiolabeled DNA molecule, a radiolabeled RNA molecule, a radiolabeled peptide, a radiolabeled polypeptide, a radiolabeled hormone, and a radiolabeled protein.

5. The method according to Claim 1 wherein the radiolabel of the radiolabeled biological molecule is selected from the group consisting of $^{125}I$, $^{131}I$, $^{90}Y$, $^{64}Cu$ and $^{212}Bi$.

6. The method according to Claim 1 wherein the ion exchange resin is an anion exchange resin.

7. The method according to Claim 6 wherein the anion exchange resin is DEAE-Sephadex.

8. The method according to Claim 1 wherein the radiolabeled biological molecule is dissolved in a buffer solution and then contacted with the ion exchange resin.

9. The method according to Claim 8 wherein the buffer solution is phosphate buffered saline.

10. The method according to Claim 8 wherein the concentration of the buffer in the buffer solution ranges from about 10 mM to about 50 mM.

11. The method according to Claim 8 wherein the pH of the buffer solution ranges from about 6·5 to about 7·5.

12. The method according to Claim 8 wherein the volume of the buffer solution ranges from about 50% to about 90% of the volume of the ion exchange resin.

13. The method according to Claim 12 wherein the buffer volume is about 80% of the volume of the ion exchange resin.

14. The method according to Claim 1 further comprising recovering the radiolabeled biological molecule from the ion exchange resin.

15. The method according to Claim 14 wherein the radiolabeled biological molecule is recovered from the ion exchange resin by eluting with a buffer solution.

FIG. 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 031 121 (NEW ENGLAND NUCLEAR CORPORATION) | 1-15 | A61K49/02 |
| Y | | 1-15 | A61K43/00 |
| | * the whole document * | | |
| P,Y | WO-A-9 105 570 (MALLINCKRODT) * page 4, line 13 - page 5, line 2; claims 1-10 * | 1-15 | |
| A | EP-A-0 250 966 (UNIVERSITY OF CINCINNATI) * the whole document * | 1-15 | |

TECHNICAL FIELDS SEARCHED (Int. Cl.5)

A61K

The present search report has been drawn up for all claims

| Place of search | Date of completion of the search | Examiner |
|---|---|---|
| THE HAGUE | 25 AUGUST 1992 | SITCH W.D.C. |